# EUROPEAN PATENT SPECIFICATION

(11) **EP 3 312 174 B1**
(45) Date of publication and mention of the grant of the patent: **18.12.2019**
(21) Application number: 15893755.7
(22) Date of filing: 05.06.2015
(51) Int. Cl.: C07D 403/10

(54) **METHOD FOR PREPARING TRITYL CANDESARTAN**
VERFAHREN ZUR HERSTELLUNG VON TRITYLCANDESARTAN
PROCÉDÉ DE PRÉPARATION DE TRITYL CANDÉSARTAN

(43) Date of publication of application: 25.04.2018
(73) Proprietor: Zhejiang Huahai Pharmaceutical Co., Ltd, Zhejiang 317024 (CN); Zhejiang Huahai Licheng Pharmaceutical Co., Ltd, Zhejiang 317024 (CN); Zhejiang Huahai Jiancheng Pharmaceutical Co., Ltd, Zhejiang 317024 (CN)
(72) Inventor: LIN, Enmin, Zhejiang 317024 (CN); MOU, Mengjian, Zhejiang 317024 (CN); TU, Guoliang, Zhejiang 317024 (CN); HUANG, Wenfeng, Zhejiang 317024 (CN)
(74) Representative: IPAZ
(86) International application number: PCT/CN2015/080867
(87) International publication number: WO 2016/192099

(56) References cited:
- WO-A1-2009/007986
- WO-A2-2008/044244
- CN-A- 1 203 223
- CN-A- 103 304 543
- CN-A- 103 396 406
- CN-A- 103 396 406

## Description

### FIELD OF THE INVENTION

The present invention relates to the field of synthesizing drug intermediates, in particular to a method for preparing trityl candesartan.

### BACKGROUND OF THE INVENTION

Candesartan cilexetil is an antihypertensive drug, which has a structural formula represented by the following formula I.

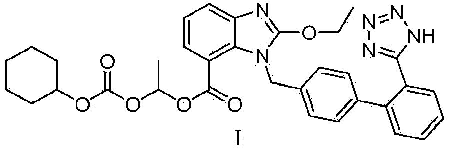

Because candesartan cilexetil does not inhibit kininase II, it does not affect the response to bradykinin. It is an antihypertensive drug having good market prospects.

Both of CN91102569.3 and CN98101894.7 report a synthetic route of candesartan cilexetil, which comprises the following steps:
a) reacting candesartan cyclic compound (II) with trialkyl tin azide, cooling the reaction solution after the end of the reaction, concentrating under reduced pressure, adding ethanol and sodium nitrite solution into the residues, adjusting pH of the solution to 4.5-5.5 by adding concentrated hydrochloric acid, adding ethyl acetate into the solution, adjusting the pH of the solution to 0.5-1.5 by adding concentrated hydrochloric acid, adding hexane into the solution, adjusting the pH of the solution to 3.5±0.5 by adding sodium hydroxide solution, and then cooling the solution to a temperature of 10 °C or below, stirring, and crystallizing to obtain an ester of candesartan (III) after separation and purification;
b) adding sodium hydroxide solution into the ester of candesartan (III) separated in step a), heating to hydrolyze, cooling the reaction solution after the end of the reaction, washing with an organic solvent, separating an aqueous layer and extracting with methanol, adding concentrated hydrochloric acid to adjust the pH to 7.0±0.5, adding active carbon and filtering, and then adding concentrated hydrochloric acid to precipitate candesartan (IV), separating and purifying candesartan (IV);
c) reacting the candesartan (IV) separated in step a) with triphenyl chloromethane in the presence of triethylamine as a catalyst, and crystallizing in hexane to obtain trityl candesartan (V);
d) reacting trityl candesartan (V) with 1-haloethylcyclohexyl carbonate to obtain trityl candesartan cilexetil (VI); and
e) deprotecting trityl candesartan cilexetil (VI) to obtian candesartan cilexetil (I); and the synthetic route is shown as follows:

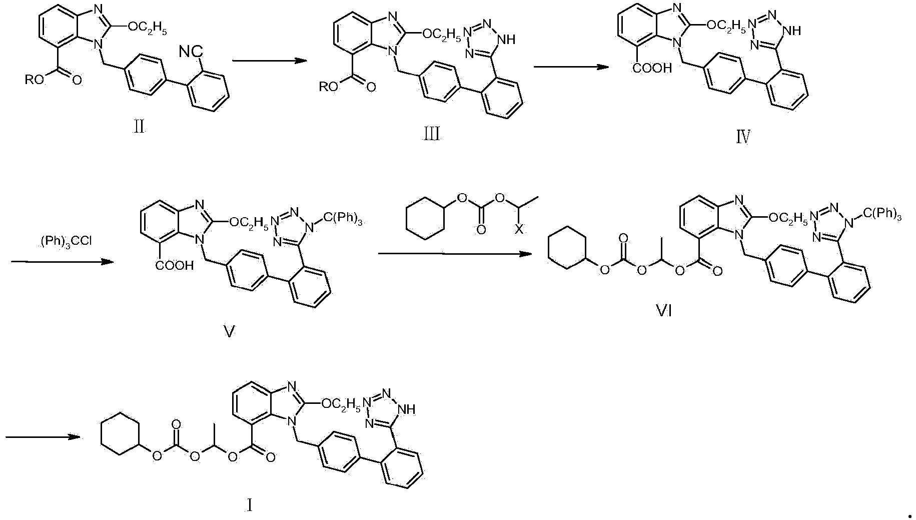

However, during the preparation of trityl candesartan (V) by using the methods described in the prior art, there are mainly following problems, for example: (1) after obtaining the ester of candesartan (III) by reacting candesartan cyclic compound (II) with trialkyl tin azide, several complex steps, such as adjusting the reaction solution to be acid, cooling, crystallizing in hexane, separating, and purifying, are required before the following reactions; and (2) after hydrolyzing the ester of candesartan (III) with sodium hydroxide solution and adjusting the solution to be acid to precipitate candesartan (IV), separating and purifying the precipitated candesartan (IV) is required before the following reactions. It can be seen that, the methods for preparing trityl candesartan (V) described in the prior art are complex, and require crystallization, separation and purification. Meanwhile, the separated crystallization mother liquor will produce a large amount of waste, which is unfavorable for green production.

### SUMMARY OF THE INVENTION

The present invention provides a method for preparing trityl candesartan (V), comprising the following steps:
(a) reacting candesartan cyclic compound (II) with trialkyl tin azide in an organic solvent to obtain an ester of candesartan (III);
(b) extracting by adding an aqueous solution of alkali metal hydroxide directly into the reaction mixture of step (a) without separating the ester of candesartan (III) obtained in step (a), and removing the organic layer to obtain an alkaline aqueous layer;
(c) heating the alkaline aqueous layer separated in step (b) to completely hydrolyze the ester of candesartan (III), and then adding an acid to adjust pH value to precipitate candesartan (IV);
(d) redissolving the candesartan (IV) by adding an organic solvent and an organic base directly into the mixture of step (c) without separating the candesartan (IV), and separating the organic layer; and
(e) reacting the organic layer obtained in step (d) directly with triphenyl chloromethane to obtain trityl candesartan (V); and
the synthetic route is shown as follows: 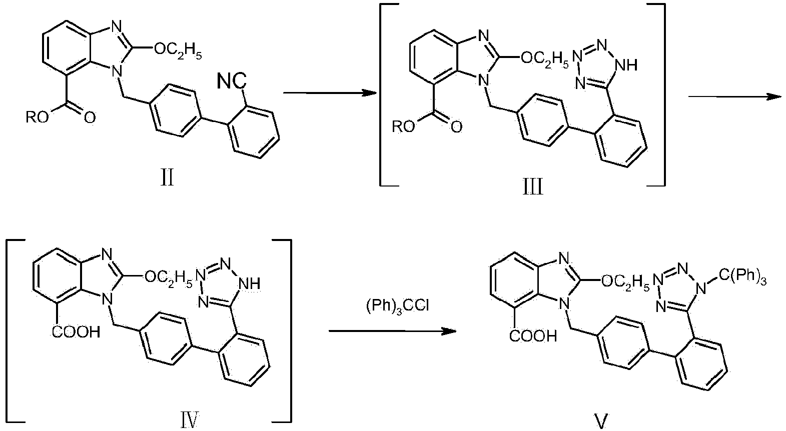 wherein, R is methyl or ethyl, preferably ethyl.

The preferred embodiments of the present invention are as follows.

The organic solvent in step (a) is preferably selected from toluene, xylene, N,N-dimethylformamide, or N,N-dimethylacetamide.

The trialkyl tin azide in step (a) is preferably tributyl tin azide.

The alkali metal hydroxide in step (b) is selected from potassium hydroxide or sodium hydroxide, further preferably sodium hydroxide.

The acid in step (c) is selected from hydrochloric acid, acetic acid or a mixture of them, further preferably acetic acid.

The pH value in step (c) is preferably in a range of 4-7, further preferably in a range of 5-6.

The organic solvent in step (d) is selected from toluene, xylene, or dichloromethane, further preferably dichloromethane.

The organic base in step (d) is preferably triethylamine.

The present invention uses candesartan cyclic compound as a starting material and employs a one-pot method to directly obtain trityl candesartan by a three-step reaction of forming tetrazole, hydrolysis, and adding a protecting group, without the steps of crystallization, separation and purification of intermediate products. The process of the present invention is simple, and is suitable for industrialized production. Moreover, according to the method for preparing trityl candesartan of the present invention, the energy consumption is reduced, the cost is saved, and the waste discharge is decreased, which are favorable for green production. Therefore, the method of the present invention is environmentally friendly.

In addition, in the methods described in the prior art, with respect to the two-step reaction of preparing candesartan by the hydrolysis of the ester of candesartan, and preparing trityl candesartan from candesartan, the acidity and basicity of the reaction systems, and the by-products from the reactions and the like are quite different, due to their different reaction conditions, such as reaction temperatures, reaction time, and solvents, etc. Therefore, the conventional processes in the prior art are adding an acid to the precipitated candesartan from the reaction solution after hydrolyzing with alkali metal hydroxide, separating and purifying it for subsequent reaction for the purpose of improving the yield and purity. However, the inventors of the present invention do not follow these conventional processes, but directly use precipitated candesartan together with the mother liquor to the following reactions, without separating it from the reaction mixture after hydrolyzing and acidifying. In this context, the inventors surprisingly find out that the yield is not decreased but significantly increased by using the method of the present invention, wherein candesartan is directly used for the following reactions without separation. Meanwhile, the method of the present invention also reduces waste discharge, and thus is favorable for green production.

Furthermore, in step (b) of the present invention, an aqueous solution of alkali metal hydroxide is added into the reaction mixture; and in step (c) of the present invention, an acid is added into the reaction mixture to precipitate candesartan. In this context, those skilled in the art cannot anticipate the influence of adding an organic base directly into such reaction system, wherein inorganic base is firstly added and then an acid is added. In addition, those skilled in the art cannot anticipate whether or not the subsequent reaction can proceed successfully, and how it proceeds. However, without being bound to any theory, the inventors of the present invention surprisingly find out that adding an organic base directly into candesartan together with the mother liquor without separating the precipitated candesartan between step (c) and step (d) not only has no negative influence on the reaction from candesartan to trityl candesartan, but also promotes the reaction. As a result, the total yield of the whole reaction is significantly increased.

### DETAILED DESCRIPTION OF THE INVENTION

The present invention is further described in detail by way of examples for better understanding of the objects, technical solution and advantages of the present invention. It is obvious that the examples described are only a part of the examples of the present invention, not all of the examples. Based on the examples of the present invention, all other examples obtained by those skilled in the art without inventive efforts are within the protection scope of the present invention.

The present invention will be further described in detail below in combination with the examples.

### Example 1:

250 g of tributyl tin azide was added into 600 ml of xylene. 100 g of candesartan cyclic compound (in formula II, R is ethyl) was added, heated to 140-150 °C, and refluxed to react for 20 h. After the end of the reaction, the reaction mixture was cooled to 40-50 °C. 600 ml of sodium hydroxide solution (48 g of sodium hydroxide dissolved in 600 ml of water) was added, and stirred under 20-35 °C. The organic layer was removed.

The alkaline aqueous layer was heated to 70-80 °C to completely hydrolyze candesartan ethyl ester. The temperature of the mixture was controlled at 25-35 °C. 400 ml of dichloromethane was added. Glacial acetic acid was added dropwise to adjust pH of the mixture to 5-6 to precipitate candesartan.

Triethylamine was added dropwise into the mixture until the candesartan solid was dissolved completely. The dichloromethane layer was separated. The aqueous layer was extracted by adding 200 ml of dichloromethane once again. The organic layers were combined. 68 g of triphenyl chloromethane was added into the organic layer. The temperature of the mixture was controlled at 25-35 °C to react until the content of candesartan was reduced to less than 1.0% monitored by HPLC. After the end of the reaction, 100 ml of water was added for washing. The aqueous layer was removed. The organic layer was dried under reduced pressure. 600 ml of anhydrous ethanol was added to crystallize. The resulting crystals were collected by filtration, and dried to provide 125.5 g of trityl candesartan, yield 78.2%, purity 97.5%.

### Example 2-7

Trityl candesartan was prepared from candesartan cyclic compound in the similar manner as Example 1. The results are shown in the following table.

| Example No. | Group R in formula II | Organic solvent in step (a) | Trialkyl tin azide in step (a) | Alkali metal hydroxide in step (b) | Acid in step (c) | Range of pH in step (c) | Organic solvent in step (d) | Organic base in step (d) | Yield |
|---|---|---|---|---|---|---|---|---|---|
| 2 | methyl | toluene | tributyl tin azide | sodium hydroxide | hydrochloric acid | 4-5 | toluene | triethylamine | 77.3% |
| 3 | ethyl | xylene | tributyl tin azide | potassium hydroxide | glacial acetic acid | 5-6 | xylene | triethylamine | 77.6% |
| 4 | methyl | DMF | tributyl tin azide | sodium hydroxide | Hydrochloric acid | 6-7 | dichloromethane | triethylamine | 78.1% |
| 5 | ethyl | DMA | tributyl tin azide | potassium hydroxide | glacial acetic acid | 4-5 | toluene | triethylamine | 77.1% |
| 6 | methyl | toluene | tributyl tin azide | sodium hydroxide | Hydrochloric acid | 5-6 | xylene | triethylamine | 77.9% |
| 7 | ethyl | xylene | tributyl tin azide | potassium hydroxide | glacial acetic acid | 6-7 | dichloromethane | triethylamine | 77.8% |

### Comparative Example 1

Trityl candesartan was prepared from candesartan cyclic compound according to the method described in Reference Example 7-9 of CN98101894.7. Since the product of the first step of the reaction in this document (corresponding to Reference Example 7) was not dried, thus the yield thereof was not calculated. The yield of the second step of the reaction was 80%, and the yield of the third step of the reaction was 89%. Therefore, the final yield of the prepared trityl candesartan from candesartan cyclic compound according to the method of this prior art was up to 73%. Besides, for example, the conversion rate of the first step of the reaction and the like were not taken into account for the above final yield. Therefore, the final yield of the method described in this document should be much less than 73%. In contrast, the one-pot method of the present invention achieves a yield of 77% or above, which is significantly higher than that of the methods of the prior art. Meanwhile, complex processes such as crystallization, separation, and purification etc. are not required, and the waste discharge is reduced.

### Comparative Example 2

250 g of tributyl tin azide was added into 600 ml of xylene. 100 g of candesartan cyclic compound (in formula II, R is ethyl) was added, heated to 140-150 °C, and refluxed to react for 20 h. After the end of the reaction, the reaction system was cooled to 40-50 °C. 600 ml of sodium hydroxide solution (48 g of sodium hydroxide dissolved in 600 ml of water) was added, and stirred under 20-35 °C. The organic layer was removed.

The alkaline aqueous layer was heated to 70-80 °C to completely hydrolyze candesartan ethyl ester. The temperature of the mixture was controlled at 25-35 °C. 400 ml of dichloromethane was added. Glacial acetic acid was added dropwise to adjust pH of the mixture to 5-6 to precipitate candesartan. The crystal was separated, washed with water and then with acetone, and dried to provide 77.6 g of candesartan (yield: 75%).

The candesartan obtained above was suspended in dichloromethane. Triethylamine was added dropwise into the mixture until the candesartan solid was dissolved completely. The dichloromethane layer was separated. The aqueous layer was extracted by adding 200 ml of dichloromethane once again. The organic layers were combined. 68 g of triphenyl chloromethane was added into the organic layer. The temperature of the mixture was controlled at 25-35 °C to react until the content of candesartan was reduced to less than 1.0% monitored by HPLC. After the end of the reaction, 100 ml of water was added for washing. The aqueous layer was removed. The organic layer was dried under reduced pressure. 600 ml of anhydrous ethanol was added to crystallize. The resulting crystals were collected by filtration, and dried to provide 120.3 g of trityl candesartan, yield 88.2%. Therefore, the final yield of this comparative example is 66.15%. In contrast, using the one-pot method of the present invention achieves a yield of 77% or above, which is significantly higher than that of the method of this comparative example. Meanwhile, complex processes such as crystallization, separation, and purification, etc. are not required, and the waste discharge is reduced.

The examples described above are only preferred examples of the present invention, and are not intended to limit the invention.

## Claims

1. A method for preparing trityl candesartan (V), comprising the following steps:
(a) reacting candesartan cyclic compound (II) with trialkyl tin azide in an organic solvent to obtain an ester of candesartan (III);
(b) extracting by adding an aqueous solution of alkali metal hydroxide directly into the reaction mixture of step (a) without separating the ester of candesartan (III) obtained in step (a), and removing the organic layer to obtain an alkaline aqueous layer;
(c) heating the alkaline aqueous layer separated in step (b) to completely hydrolyze the ester of candesartan (III), and then adding an acid to adjust pH value to precipitate candesartan (IV);
(d) redissolving the candesartan (IV) by adding an organic solvent and an organic base directly into the mixture of step (c) without separating the candesartan (IV), and separating the organic layer; and
(e) reacting the organic layer obtained in step (d) directly with triphenyl chloromethane to obtain trityl candesartan (V); and
the synthetic route is shown as follows: 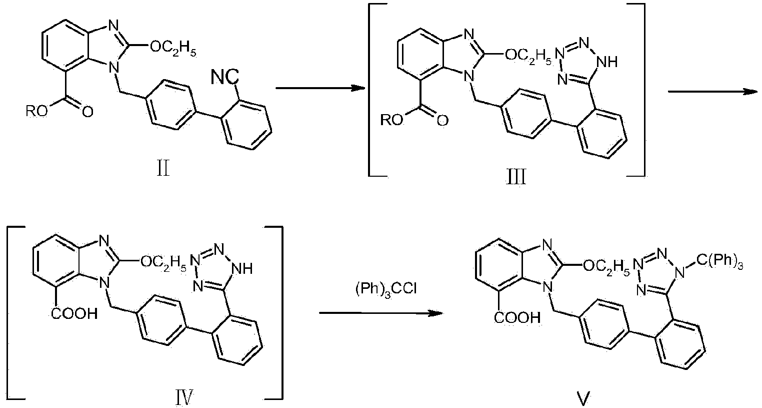 wherein, R is methyl or ethyl.

2. The method according to claim 1, wherein the organic solvent in step (a) is selected from toluene, xylene, N,N-dimethylformamide, or N,N-dimethylacetamide.

3. The method according to claim 1 or 2, wherein the trialkyl tin azide in step (a) is tributyl tin azide.

4. The method according to any one of claims 1-3, wherein the alkali metal hydroxide in step (b) is selected from potassium hydroxide or sodium hydroxide.

5. The method according to any one of claims 1-4, wherein the acid in step (c) is selected from hydrochloric acid, acetic acid or a mixture of them.

6. The method according to claim 5, wherein the acid in step (c) is acetic acid.

7. The method according to any one of claims 1-6, wherein pH value in step (c) is in a range of 4-7.

8. The method according to claim 7, wherein the pH value in step (c) is in a range of 5-6.

9. The method according to any one of claims 1-8, wherein the organic solvent in step (d) is selected from toluene, xylene, or dichloromethane.

10. The method according to claim 9, wherein the organic solvent in step (d) is dichloromethane.

11. The method according to any one of claims 1-10, wherein the organic base in step (d) is triethylamine.

## Patentansprüche

1. Verfahren zur Herstellung von Tritylcandesartan (V), umfassend die folgenden Schritte:
(a) Reagieren lassen einer Candesartan-Ringverbindung (II) mit Trialkylzinnazid in einem organischen Lösungsmittel, um einen Ester von Candesartan (III) zu erhalten;
(b) Extrahieren durch Hinzugeben einer wässrigen Lösung von Alkalimetallhydroxid direkt in das Reaktionsgemisch von Schritt (a) ohne den in Schritt (a) erhaltenen Ester von Candesartan (III) abzuscheiden, und Entfernen der organischen Schicht, um eine wässrige Alkalischicht zu erhalten;
(c) Erwärmen der in Schritt (b) abgeschiedenen wässrigen Alkalischicht, um den Ester von Candesartan (III) vollständig zu hydrolysieren, und dann Hinzugeben einer Säure zum Einstellen des pH-Wertes, um Candesartan (IV) auszufällen;
(d) wieder Auflösen des Candesartans (IV) durch Hinzugeben eines organischen Lösungsmittels und einer organischen Base direkt in die Mischung von Schritt (c) ohne Abscheiden des Candesartans (IV), und Abscheiden der organischen Schicht; und
(e) Reagieren lassen der in Schritt (d) erhaltenen organischen Schicht direkt mit Triphenylchlormethan, um Tritylcandesartan (V) zu erhalten; und
der Syntheseweg wird folgendermaßen gezeigt: 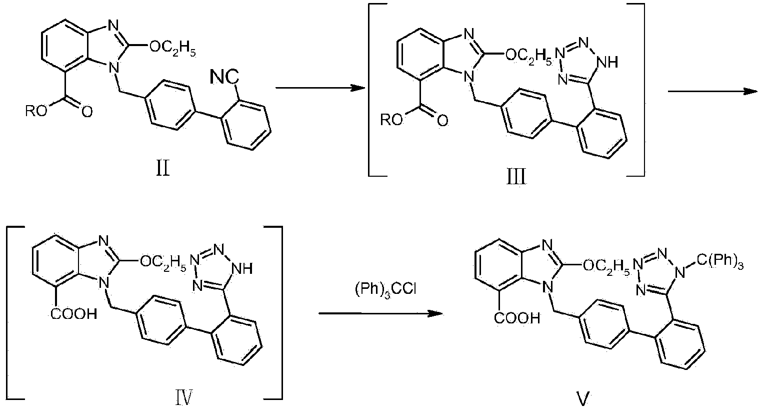 wobei R Methyl oder Ethyl ist.

2. Verfahren gemäß Anspruch 1, wobei das organische Lösungsmittel in Schritt (a) aus Toluol, Xylol, N,N-Dimethylformamid oder N,N-Dimethylacetamid ausgewählt ist.

3. Verfahren gemäß Anspruch 1 oder 2, wobei das Trialkylzinnazid in Schritt (a) Tributylzinnazid ist.

4. Verfahren gemäß einem der Ansprüche 1-3, wobei das Alkalimetallhydroxid in Schritt (b) aus Kaliumhydroxid oder Natriumhydroxid ausgewählt ist.

5. Verfahren gemäß einem der Ansprüche 1-4, wobei die Säure in Schritt (c) aus Chlorwasserstoffsäure, Essigsäure oder einer Mischung daraus ausgewählt ist.

6. Verfahren gemäß Anspruch 5, wobei die Säure in Schritt (c) Essigsäure ist.

7. Verfahren gemäß einem der Ansprüche 1-6, wobei der pH-Wert in Schritt (c) im Bereich von 4-7 liegt.

8. Verfahren gemäß Anspruch 7, wobei der pH-Wert in Schritt (c) im Bereich von 5-6 liegt.

9. Verfahren gemäß einem der Ansprüche 1-8, wobei das organische Lösungsmittel in Schritt (d) aus Toluol, Xylol oder Dichlormethan ausgewählt ist.

10. Verfahren gemäß Anspruch 9, wobei das organische Lösungsmittel in Schritt (d) Dichlormethan ist.

11. Verfahren gemäß einem der Ansprüche 1-10, wobei die organische Base in Schritt (d) Triethylamin ist.

## Revendications

1. Procédé de préparation du trityl-candésartan (V), comprenant les étapes suivantes :
(a) réaction d'un dérivé cyclique du candésartan (II) avec un azoture de trialkylétain dans un solvant organique pour obtenir un ester de candésartan (III) ;
(b) extraction par addition d'une solution aqueuse d'un hydroxyde d'un métal alcalin directement dans le mélange réactionnel de l'étape (a) sans séparation de l'ester de candésartan (III) obtenu à l'étape (a) et élimination de la couche organique pour obtenir une couche aqueuse alcaline ;
(c) chauffage de la couche aqueuse alcaline séparée à l'étape (b) pour hydrolyser entièrement l'ester de candésartan (III), puis addition d'un acide pour ajuster la valeur du pH de manière à faire précipiter le candésartan (IV) ;
(d) redissolution du candésartan (IV) par addition d'un solvant organique et d'une base organique directement dans le mélange de l'étape (c) sans séparation du candésartan (IV) et séparation de la couche organique ; et
(e) réaction de la couche organique obtenue à l'étape (d) directement avec du triphénylchlorométhane pour obtenir le trityl-candésartan (V) ; et
la voie de synthèse étant représentée comme suit : 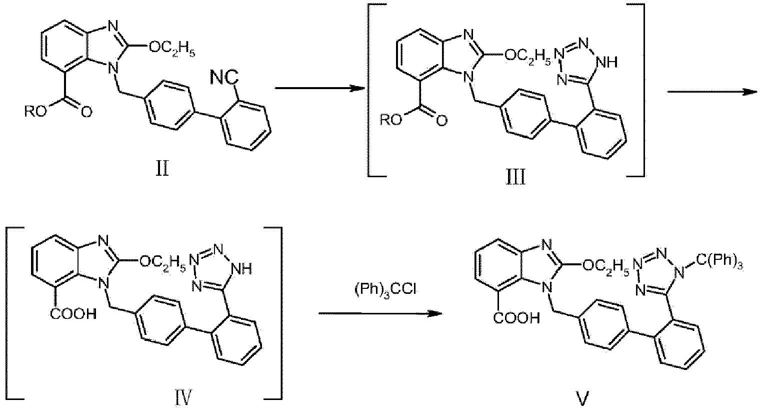 où R est un méthyle ou un éthyle.

2. Procédé selon la revendication 1, où le solvant organique de l'étape (a) est sélectionné parmi le toluène, le xylène, le N,N-diméthylformamide ou le N,N-diméthylacétamide.

3. Procédé selon la revendication 1 ou 2, où l'azoture de trialkylétain de l'étape (a) est l'azoture de tributylétain.

4. Procédé selon l'une quelconque des revendications 1-3, où l'hydroxyde d'un métal alcalin de l'étape (b) est sélectionné parmi l'hydroxyde de potassium ou l'hydroxyde de sodium.

5. Procédé selon l'une quelconque des revendications 1-4, où l'acide de l'étape (c) est sélectionné parmi l'acide chlorhydrique, l'acide acétique ou un mélange de ceux-ci.

6. Procédé selon la revendication 5, où l'acide de l'étape (c) est l'acide acétique.

7. Procédé selon l'une quelconque des revendications 1-6, où la valeur du pH à l'étape (c) est dans une plage de 4-7.

8. Procédé selon la revendication 7, où la valeur du pH à l'étape (c) est dans une plage de 5-6.

9. Procédé selon l'une quelconque des revendications 1-8, où le solvant organique de l'étape (d) est sélectionné parmi le toluène, le xylène ou le dichlorométhane.

10. Procédé selon la revendication 9, où le solvant organique de l'étape (d) est le dichlorométhane.

11. Procédé selon l'une quelconque des revendications 1-10, où la base organique de l'étape (d) est la triéthylamine.
